# EUROPÄISCHE PATENTSCHRIFT

(11) **EP 0 601 328 B1**
(45) Veröffentlichungstag und Bekanntmachung des Hinweises auf die Patenterteilung: **17.12.1997**
(21) Anmeldenummer: 93117572.3
(22) Anmeldetag: 28.10.1993
(51) Int. Cl.: A61N 1/05, A61N 1/39

(54) **Defibrillierungssystem**
Defibrillation system
Système de defibrillation

(30) Priorität: 11.12.1992 SE 9203734
(43) Veröffentlichungstag der Anmeldung: 15.06.1994
(73) Patentinhaber: Pacesetter AB, 171 95 Solna (SE)
(72) Erfinder: Ljungström, Jan, S-171 40 Solna (SE)
(74) Vertreter: Lettström, Richard Wilhelm

(56) Entgegenhaltungen:
- EP-A- 0 500 289
- US-A- 3 937 225
- US-A- 5 010 894

## Beschreibung

Die Erfindung betrifft ein Defibrillierungssystem gemäß Anspruch 1.

Ein Defibrillierungssystem dieser Art ist durch die US-PS 4 662 377 bekannt. An den Defibrillator sind zwei Defibrillierungselektroden angeschlossen, bei denen die eine Elektrode subkutan und die andere Elektrode intrakardial appliziert ist. Der Elektrodenkopf der intrakardial applizierten Defibrillierungselektrode weist einen wendelförmigen Leiter auf, dessen Durchmesser etwa dem Aussendurchmesser des Elektrodenkabels entspricht, wobei der Leiter einen längeren Teil des distalen Endes der Elektrode entlang verläuft. Der Nachteil einer solchen Defibrillierungselektrode ist, dass deren Defibrillierungsoberfläche im Vergleich zur Grösse desjenigen Stroms, der dieser Oberfläche beim Abgeben eines Defibrillierungsimpulses zugeführt wird, verhältnismässig klein ist. Da die Defibrillierungsoberfläche meistens gegen die Herzwand anliegt, können Brandwunden am Herzen entstehen. Durch die Grösse, die Applizierung und die Konzentration der Defibrillierungsoberfläche an einer Stelle im Herzen wird keine optimale Defibrillierung erhalten.

In der US-PS 5 010 894 ist eine Defibrillierungselektrode beschrieben, die für eine intrakardiale Plazierung vorgesehen ist. Der Elektrodenkopf umfasst eine Anzahl nach aussen gerichtete, vorgeformte flexible Leiter, die als Defibrillierungsoberflächen dienen, wobei die proximalen Enden der Leiter nebeneinander an einem gemeinsamen Verbindungselement befestigt sind gleichzeitig damit, dass deren distale Enden nebeneinander an einem weiteren gemeinsamen Verbindungselement fest angebracht sind. Bevor eine Einführung des Elektrodenkabels in das Herz über eine Vene erfolgt, wird der Elektrodenkopf mit Hilfe eines Mandrins derart gestreckt, dass die Leiter dicht nebeneinander zu liegen kommen, wobei der Aussendurchmesser des Elektrodenkopfes lediglich etwas grösser als der Aussendurchmesser des Elektrodenkabels ist. Nachdem der Elektrodenkopf in das Herz eingeführt ist, kann der Mandrin herausgezogen werden. Dabei expandieren die Leiter seitlich, so dass sie über eine erhebliche Strecke der Länge federnd gegen die umgebende Wand zu liegen kommen. Durch diese Defibrillierungselektrode kann nun der Strom, der den Leitern zugeführt wird, gleichmässig verteilt werden, wobei die Leiter zusammen eine verhältnismässig grosse Defibrillierungsoberfläche bilden. Dies kann verhindern, dass Brandwunden an der umgebenden Herzwand entstehen. Dadurch, dass auch die Leiter im Herz gleichmässig verteilt werden, kann auch eine optimale Defibrillierung erreicht werden. Da der Elektrodenkopf im Herzen permanent angebracht ist, müssen wenigstens die Leiter extrem gute Materialeigenschaften aufweisen, damit diese während einer längeren Zeit den Bewegungen des Herzens folgen können,ohne dass sie oder das Herz zu Schaden kommen. Ausserdem kann ein derartiger verhältnismässig grosser Elektrodenkopf den Blutfluss im Herzen behindern.

In der EP-A-500 289 ist ein Defibrillierungssystem beschrieben, das den Oberbegriff des Anspruchs 1 entspricht.

Der Erfindung liegt die Aufgabe zugrunde, ein Defibrillierungssystem mit einer intrakardialen Defibrillierungselektrode der eingangs genannten Art zu schaffen, deren Defibrillierungsoberfläche verhältnismässig gross ist und im Herzen derart verteilt werden kann, dass Schäden am Herzen vermieden werden gleichzeitig damit, dass eine optimale Defibrillierung erhalten werden kann. Ausserdem soll die Defibrillierungselektrode in dem Zeitraum, in dem sie keine Stimulationsimpulse abgibt, den Blutfluss im Herzen nicht beeinflussen.

Diese Aufgabe ist erfindungsgemäss durch die kennzeichnenden Merkmale des Anspruchs 1 gelöst. Wenn die Kontrollmittel, die den Zustand des Herzens abtasten, den Bedarf einer Defibrillierung des Herzens anzeigen, expandiert der Elektrodenkopf mit Hilfe der erwähnten Steuermittel,so dass die Defibrillierungsoberfläche oder die Flächen gegen die umgebende Herzwand oder in die Nähe der umgebenden Herzwand zu liegen kommen, wonach dem Herzen Defibrillierungsimpulse zugeführt werden. Wenn das Herz danach in einen Normalzustand zurückgeht, wird der Elektrodenkopf mittels der Steuermittel in seine ursprüngliche Lage zurückgeführt. In dieser Lage weist der Elektrodenkopf eine Form auf, die den Blutfluss im Herzen nicht beeinflusst. Wenn der Elektrodenkopf in eine solche passive Lage gebracht ist, kann weder der Elektrodenkopf noch die umgebende Herzwand zu Schaden kommen. Die Form des Elektrodenkopfes in einer passiven Lage ähnelt einem schlauchförmigen Körper, der eine Verlängerung des Elektrodenkabels in dessen Längsrichtung bildet und der etwa im Zentrum der Herzkammer angebracht ist.

In einer Weiterbildung der Erfindung wird vorgeschlagen, dass der Elektrodenkopf der Defibrillierungselektrode aus einem aufblasbaren Material, das vorzugsweise elastisch ist, besteht. Hierdurch ist erreicht, dass der Elektrodenkopf im aufgeblasenen Zustand gegen einen grossen Teil der umgebenden Herzwand anliegen kann.

In einer vorteilhaften Ausbildung der Erfindung wird vorgeschlagen, dass die Defibrillierungsoberflächen am aufblasbaren Material aus drahtförmigen Leitern gebildet sind, die derart angeordnet sind, dass sie eine Expandierung des Elektrodenkopfes erlauben. Die Leiter sind vorzugsweise über den Elektrodenkopf gleichmässig verteilt. Durch Wahl der Anzahl der Leiter und Wahl der Grösse der Leiter kann eine gewünschte Grösse der Defibrillierungsoberfläche erhalten werden.

Nach einer weiteren Ausgestaltung des Elektrodenkopfes der Defibrillierungselektrode besteht dieser aus mindestens zwei langgestreckten flexiblen schenkelförmigen Teilen, die ganz oder teilweise als Defibrillierungsoberflächen dienen, deren eine Enden am Elektrodenkabel befestigt sind, und die mindestens teilweise gegeneinander liegen, wobei die schenkelförmigen Teile mittels eines Trennorgans expandierbar sind. Durch diesen Aufbau kann jedes schenkelförmige Teil eine verhältnismässig grosse Defibrillierungsoberfläche aufweisen. Ausserdem kann der Elektrodenkopf bei einer Defibrillierung abhängig von der Länge und der Anzahl der schenkelförmigen Teile gegen den grösseren Teil der umgebenden Herzwand in der Kammer anliegen.

Das Trennorgan kann nach der Erfindung vorzugsweise ein aufblasbares ballonähnliches Teil sein, das an das Elektrodenkabel angeschlossen und zwischen den schenkelförmigen Teilen angebracht ist.

Eine konstruktiv einfache Ausgestaltung der Erfindung wird erhalten, in dem die Steuermittel eine Pumpe sind, die über einen Kanal im Elektrodenkabel dem Elektrodenkopf bzw. dem ballonähnlichen Teil ein Gas oder eine Flüssigkeit zuführt bzw. über den Kanal ein Gas oder eine Flüssigkeit vom Elektrodenkopf bzw. vom ballongähnlichen Teil entfernt.

Im Rahmen der Erfindung wird auch vorgeschlagen, dass die Kontrollmittel die Steuermittel derart beeinflussen, dass eine Expandierung des Elektrodenkopfes erfolgt, bevor ein Defibrillierungsimpuls abgegeben wird. Auf diese Weise ist eine optimale Defibrillierung des Herzens sichergestellt.

Die Erfindung ist nachfolgend anhand mehrerer in den Zeichnungen dargestellten Ausführungsbeispielen näher erläutert. Es zeigen:
- FIG 1: ein Defibrillierungssystem mit einer intrakardialen Defibrillierungselektrode nach der Erfindung,
- FIG 2: eine weitere Ausführungsform der Defibrillierungselektrode nach FIG 1,
- FIG 3 und 4: weitere Ausführungsformen einer intrakardialen Defibrillierungselektrode nach der Erfindung und
- FIG 5: ein Blockschaltbild eines Defibrillierungssystems nach der Erfindung.

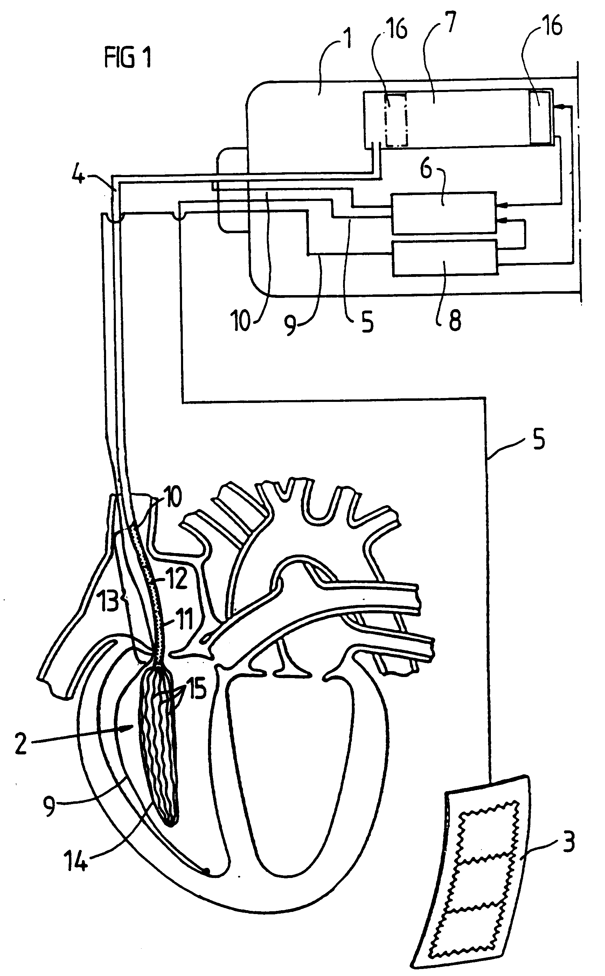

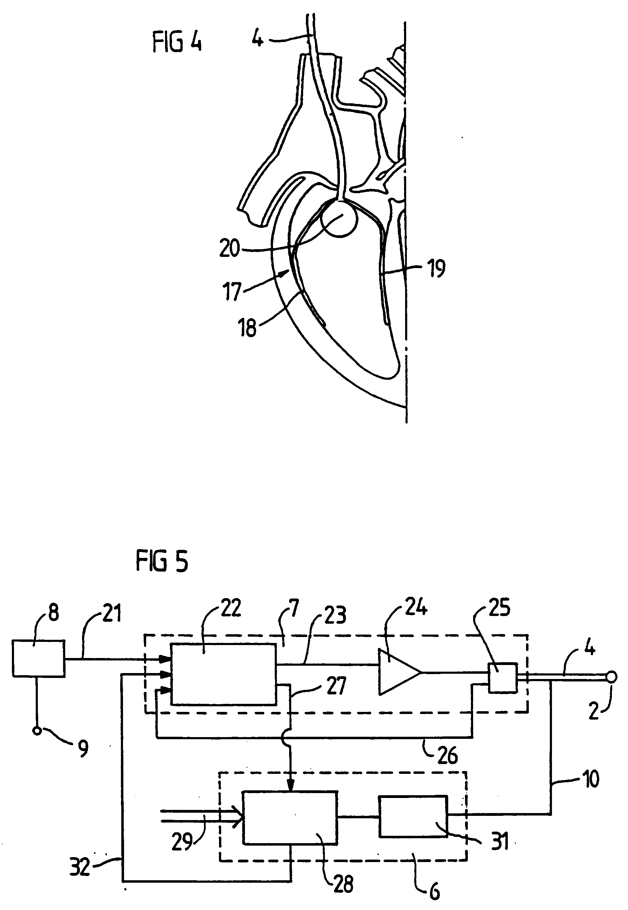

In der FIG 1 ist ein Defibrillierungssystem mit einem Defibrillator 1 gezeigt, an dem zwei Defibrillierungselektroden 2, 3 angeschlossen sind, wobei die eine Defibrillierungselektrode 2 intrakardial und die andere Defibrillierungselektrode 3 z.B. subkutan plaziert ist. Die andere Defibrillierungselektrode 3 oder eine dritte Defibrillierungselektrode in einem solchen Defibrillierungssystem kann auch im Bereich der Vena Cava Superior angebracht sein, wobei eine solche Defibrillierungselektrode eine für diesen Bereich angepasste Form aufweist. Die Defibrillierungselektroden 2, 3 sind über Elektrodenkabel 4, 5 an einen im Defibrillator 1 angebrachten Impulsgenerator 6 angeschlossen. Die intrakardial applizierte Defibrillierungselektrode 2 ist auch an Steuermittel 7 angeschlossen. Das Defibrillierungssystem umfasst auch Kontrollmittel 8, an die eine intrakardial plazierte Sensorelektrode 9 angeschlossen ist. Der erwähnte Impulsgenerator 6 sowie die Steuer- und Kontrollmittel 7, 8 werden unten in Verbindung mit dieser FIG und der FIG. 5 näher beschrieben.

Die intrakardiale Defibrillierungselektrode 2 besteht aus dem erwähnten flexiblen Elektrodenkabel 4, das einen langgestreckten Leiter 10 umfasst, dessen Aussenseite mit einer Isolierschicht 11 versehen ist und dessen Innenseite einen Kanal 12 bildet. Diese Ausführungsform wird mit Hilfe des Längsschnittes 13 am Elektrodenkabel 4 dargestellt. Am distalen Ende des Elektrodenkabels 4 ist ein Elektrodenkopf 14 aus einem aufblasbaren Material, das vorzugsweise elastisch ist, angebracht. Die Defibrillierungsoberflächen am aufblasbaren Material sind aus drahtförmigen Leitern 15 gebildet, die derart angeordnet sind, dass sie eine Expandierung des Elektrodenkopfes 14 erlauben. In diesem Ausführungsbeispiel verlaufen die drahtförmigen Leiter 15, die mit dem Leiter 10 verbunden sind, von der Befestigungsstelle am Elektrodenkabel 4 bis zum distalen Ende des Elektrodenkopfes 14 und sind dort, um Spannungsunterschiede zwischen den drahtförmigen Leitern 15 zu vermeiden, miteinander verbunden.

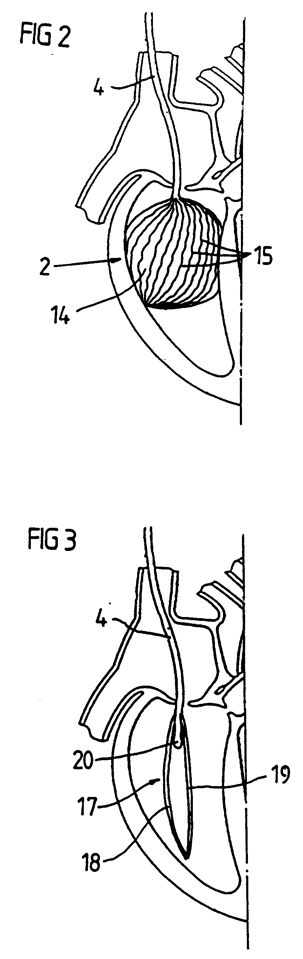

Wenn die Kontrollmittel 8 durch Signale von der Sensorelektrode 9 ein Herzflimmern anzeigen, wird diese Information den Steuermitteln 7 zugeführt, die über das Elektrodenkabel 4 den Elektrodenkopf 14 umgehend expandiert, so dass dessen Aussenwand mit den drahtförmigen Leitern 15 dicht an der umgebenden Herzwand, wie es in der FIG. 2 gezeigt ist, anliegt. Wenn die Steuermittel 7 den Elektrodenkopf 14 in diese aktive Lage gebracht haben, wird dem Impulsgenerator 6 diese Information zugeleitet, der dann über die Defibrillierungselektroden 2 und 3 Defibrillierungsimpulse ans Herz abgibt. Die Steuermittel 7 für die intrakardial plazierte Defibrillierungselektrode 2 sind eine Pumpe, z.B. eine Kolbenpumpe, die dem Elektrodenkopf 14 über den Kanal 12 des Elektrodenkabels 4 ein Gas oder eine Flüssigkeit, wie z.B. Kochsalzlösung, zuführt. Durch die in der FIG. 1 schematisch gezeigte Pumpe 7 soll dargestellt werden, dass, wenn die Defibrillierungselektrode 2 in einer in dieser FIG gezeigten Lage zusammengezogen ist, der Kolben 16 eine Position aufweist, die mit den durchgezogenen Konturenlinien gezeigt ist. Der Raum in der Pumpe 7 und auch der Kanal 12 des Elektrodenkabels 4 sind hier mit einem Gas oder einer Flüssigkeit gefüllt. Bevor eine Defibrillierung des Herzens erfolgen soll, wird der Kolben 16 in eine Lage, die durch die strichpunktierten Konturenlinien gezeigt sind, verschoben, wobei das Gas oder die Flüssigkeit zum Elektrodenkopf 14 transportiert wird, der wie in Verbindung mit der FIG. 2 beschrieben ist, expandiert. Wenn die Sensorelektrode 9 wieder eine normale Herztätigkeit anzeigt, wird dieses Ergebnis über die Kontrollmittel 8 teils dem Impulsgenerator 6, der dann die Abgabe von Defibrillierungsimpulsen einstellt und teils der Pumpe 7 zugeleitet, wobei die Pumpe 7 mit Hilfe des Kolbens 16 das Gas oder die Flüssigkeit aus den Elektrodenkopf 14 heraussaugt, so dass der Elektrodenkopf in seine ursprüngliche passive Lage zusammengezogen wird.

In der FIG. 3 ist eine intrakardiale Defibrillierungselektrode abgebildet mit einem Elektrodenkopf 17, der sich in seiner Ausführungsform von dem in den FIG 1 und 2 gezeigten Elektrodenkof unterscheidet. Dieser Elektrodenkopf 17 umfasst eine Anzahl schenkelförmige Teile 18,19, die an den hier nicht gezeigten Leiter 10 des Elektrodenkabels 4 angeschlossen sind und die als Defibrillierungsoberflächen dienen. In den FIG. 3 und 4 sind anschaulichkeitshalber lediglich zwei schenkelförmige Teile 18, 19 abgebildet. Der Elektrodenkopf 17 kann aber vorzugsweise mit einer grösseren Anzahl solcher Teile 18,19, die am distalen Ende des Elektrodenkabels gleichmässig verteilt befestigt sind, versehen werden. Die schenkelförmigen Teile 18, 19 weisen ein derartiges Material und eine derartige Form auf, dass diese, wenn sie in eine in der FIG. 3 gezeigte passive Lage gebracht werden, wenigstens teilweise gegeneinander zu liegen kommen. Am distalen Ende des Elektrodenkabels 4 zwischen den schenkelförmigen Teilen 18, 19 ist auch ein aufblasbares ballonähnliches Teil 20 befestigt, das über den Kanal des Elektrodenkabels 4 mit der Pumpe 7 verbunden ist. Bevor eine Defibrillierung erfolgen soll, wird das ballonähnliche Teil 20 in einer in Verbindung mit den FIG. 1 und 2 beschriebenen Weise aufgeblasen, so dass die schenkelförmigen Teile 18, 19 expandieren und sich gegen die umgebende Herzwand legen, wobei Defibrillierungsimpulse abgegeben werden können. Dadurch, dass das aufblasbare Teil 20 verhältnismässig klein gemacht werden kann, kann auch die Pumpe 7 entsprechend klein gehalten werden. Nach einer Defibrillierung des Herzens geht der Elektrodenkopf 17, indem die Pumpe 7 das Gas oder die Flüssigkeit aus dem Teil 20 heraussaugt, in seine ursprüngliche passive Lage zurück.

In einer weiteren nicht gezeigten Ausführungsform der Defibrillierungselektrode 2 können die Bewegungen der schenkelförmigen Teile 18,19 auch mit Hilfe eines dünnen Drahtes, der über den Kanal 12 des Elektrodenkabels 4 mit einer im Defibrillator 1 angebrachten motorgetriebenen Rolle verbunden ist und auf der der Draht auf- bzw. abgewickelt werden kann, erfolgen. Der Draht und die motorgetriebene Rolle sollen dabei die Pumpe 7 ersetzen.

In FIG 5 ist ein Blockschaltbild des beschriebenen Defibrillierungssystemsgezeigt. In diesem Ausführungsbeispiel nach FIG. 5 werden die Herzsignale von der Sensorelektrode 9 abgetastet. Diese sind auf die Kontrollmittel 8 geschaltet, die in bekannter Weise abtasten, ob Herzflimmern vorliegt. Wenn dies der Fall ist und ein Defibrillierungsschock abgegeben werden soll, wird einem Signal über eine Leitung 21 einer Erweiterungslogik 22, die ein Teil des Steuermittels 7 ist, zugeführt. Das Ausgangssignal von der Erweiterungslogik 22 steuert über eine Leitung 23 und möglicherweise über einen Verstärker 24 eine Pumpe 25, um in beschriebener Weise die Elektrode 2 über das Elektrodenkabel 4 zu erweitern. Über eine Leitung 26 wird der Status der Pumpe 25 zur Erweiterungslogik 22 zurückgeschaltet. Die Erweiterungslogik 22 stellt fest, dass die Elektrode 2 ausgeweitet ist und gibt über eine Ausgangsleitung 27 einen Steuerimpuls an die Schocklogik 28, die ein Teil des Impulsgenerators ist, ab. Parameter, wie z.B. das Energieniveau, können, wie bereits angedeutet, mittels einer Leitung 29 in der Schocklogik 28 programmiert werden. Die Schocklogik 28 erzeugt ein Ausgangssignal, das über eine Leitung 30 eine Endstufe für die Erzeugung eines Defibrillierungsimpulses steuert, der über den Leiter 10 der zur Zeit erweiterten Elektrode 2 zugeführt wird. Die Schocklogik 28 erzeugt ferner ein Signal, das anzeigt, wenn der Schock beendet ist. Dieses Signal wird über eine Leitung 32 der Erweiterungslogik 22 zugeleitet, die abhängig von den Signalen der Kontrollmittel 8 entscheidet, ob das Herzflimmern durch die Defibrillierungsimpulse unterbrochen wurde und diese Impulse das Herz zu einer normalen Aktivität zwang oder, ober Herzflimmern noch vorliegt und ein weiterer Schockimpuls abgegeben werden soll. Davon abhängig wird die Elektrode in ihrer erweiterten Lage gehalten, alternativ wird ein Signal erzeugt, das über die Pumpe 25 die Elektrode in die Form bringt, die sie haben soll, solange kein Schockimpuls zum Herz abgegeben werden soll.

## Patentansprüche

1. Defibrillierungssystem mit einem Defibrillator (1), an dem mindestens zwei Defibrillierungselektrode (2, 3) angeschlossen sind, wobei mindestens eine Defibrillierungselektrode für eine intrakardiale Plazierung vorgesehen ist, und dass diese Defibrillierungselektrode ein flexibles Elektrodenkabel (4, 5) mit mindestens einem langgestreckten elektrisch isolierten Leiter (10) und mit einem am distalen Ende des Elektrodenkabels angebrachten Elektrodenkopf (14, 17) mit mindestens einer Defibrillierungsoberfläche (15) für die Übertragung von Defibrillierungsimpulsen an das Herz umfasst und wobei das Defibrillierungssystem Kontrollmittel (8) umfasst, die entscheiden, wenn der Defibrillator eine oder mehrere Defibrillierungsimpulse abgeben soll, wobei der Elektrodenkopf expandierbar ist und Steuermittel (7) zur Expandierung des Elektrodenkopfs vorgesehen sind, **dadurch gekennzeichnet**, dass die Kontrollmittel (8) die Steuermittel (7) beeinflussen, dass die Steuermittel (7), bei Bedarf einer Defibrillierung des Herzens, den Elektrodenkopf (14,17) über das Elektrodenkabel (4,5) expandierend beeinflussen, und dass die Kontrollmittel (8) die Steuermittel (7) derart beeinflussen, dass eine Expandierung des Elektrodenkopfes (14,17) erfolgt, bevor ein Defibrillierungsimpuls abgegeben wird.

2. Defibrillierungssystem nach Anspruch 1, **dadurch gekennzeichnet**, dass der Elektrodenkopf (14) der Defibrillierungselektrode (2) aus einem aufblasbaren Material, das vorzugsweise elastisch ist, besteht.

3. Defibrillierungssystem nach Anspruch 2, **dadurch gekennzeichnet**, dass die Defibrillierungsoberflächen (15) am aufblasbaren Material aus drahtförmigen Leitern gebildet sind, die derart angeordnet sind, dass sie eine Expandierung des Elektrodenkopfes (14) erlauben.

4. Defibrillierungssystem nach Anspruch 1, **dadurch gekennzeichnet**, dass der Elektrodenkopf (17) der Defibrillierungselektrode (2) mindestens zwei langgestreckte flexible schenkelförmige Teile (18, 19) umfasst, die ganz oder teilweise als Defibrillierungsoberflächen dienen, deren eine Enden am Elektrodenkabel (4) befestigt sind, und die mindestens teilweise gegeneinander liegen, wobei die schenkelförmigen Teile (18, 19) mittels eines Trennorgans (20) expandierbar sind.

5. Defibrillierungssystem nach Anspruch 4, **dadurch gekennzeichnet**, dass das Trennorgan (20) ein aufblasbares ballonähnliches Teil ist, das an das Elektrodenkabel (4) angeschlossen und zwischen den schenkelförmigen Teilen (18, 19) angebracht ist.

6. Defibrillierungssystem nach einem der Ansprüche 2 bis 5, **dadurch gekennzeichnet**, dass die Steuermittel (7) eine Pumpe sind, die über einen Kanal (12) im Elektrodenkabel (4) dem Elektrodenkopf (14) bzw. dem ballonähnlichen Teil (17) ein Gas oder eine Flüssigkeit zuführt bzw. über den Kanal ein Gas oder eine Flüssigkeit vom Elektrodenkopf (14) bzw. vom ballongähnlichen Teil (17) entfernt.

## Claims

1. Defibrillation system having a defibrillator (1), to which are connected at least two defibrillation electrodes (2, 3), with at least one of the defibrillation electrodes being intended for intracardiac placing, and that this defibrillation electrode comprises a flexible electrode cable (4, 5) having at least one elongated electrically insulated conductor (10) and having an electrode head (14, 17) which is attached to the distal end of the electrode cable and has at least one defibrillation surface (15) for the transmission of defibrillation pulses to the heart, and with the defibrillation system comprising checking means (8), which decide when the defibrillator is to emit one or more defibrillation pulses, with the electrode head being expandable and control means (7) being provided for expanding the electrode head, characterised in that the checking means (8) influence the control means (7), in that the control means (7), if a defibrillation of the heart is required, influence the electrode head (14, 17) in an expanding manner by way of the electrode cable (4, 5), and in that the checking means (8) influence the control means (7) in such a way that an expansion of the electrode head (14, 17) takes place before a defibrillation pulse is emitted.

2. Defibrillation system according to claim 1, characterised in that the electrode head (14) of the defibrillation electrode (2) consists of an inflatable material, which is preferably elastic.

3. Defibrillation system according to claim 2, characterised in that the defibrillation surfaces (15) on the inflatable material are formed from wire-shaped conductors, which are arranged in such a way that they permit an expansion of the electrode head (14).

4. Defibrillation system according to claim 1, characterised in that the electrode head (17) of the defibrillation electrode (2) comprises at least two elongated flexible web-like portions (18, 19) which are used completely or partially as defibrillation surfaces, the one ends of which are fastened to the electrode cable (4), and which web-like portions lie at least partially against each other, with the web-like portions (18, 19) being expandable by means of a separating element (20).

5. Defibrillation system according to claim 4, characterised in that the separating element (20) is an inflatable balloon-like portion, which is connected to the electrode cable (4) and is mounted between the web-like portions (18, 19).

6. Defibrillation system according to one of the claims 2 to 5, characterised in that the control means (7) is a pump, which supplies a gas or a liquid to the electrode head (14) or to the balloon-like portion (17) by way of a channel (12) in the electrode cable (4), or removes a gas or a liquid from the electrode head (14) or from the balloon-like portion (17) by way of the said channel.

## Revendications

1. Système de défibrillation, comportant un défibrillateur (1) auquel sont raccordées au moins deux électrodes (2, 3) de défibrillation, une électrode de défibrillation au moins étant prévue pour une implantation intracardiaque, et cette électrode de défibrillation comportant un câble flexible (4, 5) d'électrode ayant au moins un conducteur oblong (10) électriquement isolé, ainsi qu'une tête (14, 17) d'électrode disposée à l'extrémité distale du câble d'électrode et ayant au moins une surface (15) de défibrillation destinée à transmettre des impulsions de défibrillation au coeur, et le système de défibrillation comportant des moyens de contrôle (8) qui décident à quel moment le défibrillateur doit émettre une ou plusieurs impulsions de défibrillation, la tête d'électrode étant expansible et il est prévu des moyens de commande (7) destinés à se rendre maître de l'expansion de la tête d'électrode, caractérisé par le fait que les moyens de contrôle (8) agissent sur les moyens de commande (7), que, lorsque le coeur a besoin d'une défibrillation, les moyens de commande (7) agissent sur la tête (14, 17) d'électrode en l'expansant par l'intermédiaire du câble (4, 5) d'électrode, et que les moyens de contrôle (8) agissent sur les moyens de commande (7) de telle sorte que la tête (14, 17) d'électrode soit expansée avant qu'une impulsion de défibrillation soit émise.

2. Système de défibrillation selon la revendication 1, caractérisé par le fait que la tête (14) de l'électrode (2) de défibrillation est constituée d'un matériau gonflable, qui est de préférence élastique.

3. Système de défibrillation selon la revendication 2, caractérisé par le fait que les surfaces (15) de défibrillation du matériau gonflable sont constituées de conducteurs filiformes, qui sont disposés de telle sorte qu'ils permettent une expansion de la tête (14) de l'électrode.

4. Système de défibrillation selon la revendication 1, caractérisé par le fait que la tête (17) de l'électrode (2) de défibrillation comporte au moins deux éléments oblongs flexibles (18, 19) en forme de branche, qui servent entièrement ou partiellement de surfaces de défibrillation, qui sont fixés par l'une de leurs extrémités au câble (4) d'électrode, et qui sont situés, au moins partiellement, l'un contre l'autre, les éléments (18, 19) en forme de branche pouvant être expansés au moyen d'un organe de séparation (20).

5. Système de défibrillation selon la revendication 4, caractérisé par le fait que l'organe de séparation (20) est un élément gonflable de type ballon qui est raccordé au câble (4) d'électrode, et qui est disposé entre les éléments (18, 19) en forme de branche.

6. Système de défibrillation selon l'une quelconque des revendications 2 à 5, caractérisé par le fait que les moyens de commande (7) sont une pompe qui amène un gaz ou un liquide à la tête (14) d'électrode ou à l'élément (20) de type ballon par l'intermédiaire d'un conduit (12) pratiqué dans le câble (4) d'électrode, ou qui évacue un gaz ou un liquide de la tête (14) d'électrode ou de l'élément (20) de type ballon par l'intermédiaire du conduit.
